# EUROPÄISCHE PATENTSCHRIFT

(11) **EP 2 205 716 B1**
(45) Veröffentlichungstag und Bekanntmachung des Hinweises auf die Patenterteilung: **27.09.2017**
(21) Anmeldenummer: 08837177.8
(22) Anmeldetag: 08.10.2008
(51) Int. Cl.: B01F 11/00, B01L 3/00, B01F 15/00, C12M 1/32, C12M 1/04, C12M 1/00

(54) **MIKROREAKTOR**
MICROREACTOR
MICRORÉACTEUR

(30) Priorität: 08.10.2007 DE 102007048201; 08.02.2008 DE 102008008256
(43) Veröffentlichungstag der Anmeldung: 14.07.2010
(73) Patentinhaber: M2p-labs GmbH, 52499 Baesweiler (DE)
(72) Erfinder: KENSY, Frank, 52064 Aachen (DE); MÜLLER, Carsten, 52134 Herzogenrath (DE); BÜCHS, Jochen, 52064 Aachen (DE); FUNKE, Matthias, 3904 Naters (CH)
(74) Vertreter: Castell, Klaus
(86) Internationale Anmeldenummer: PCT/DE2008/001623
(87) Internationale Veröffentlichungsnummer: WO 2009/046697

(56) Entgegenhaltungen:
- EP-A- 1 733 793
- EP-A- 1 944 080
- WO-A-98/45406
- WO-A-02/081075
- WO-A-02/102965
- WO-A-2005/098397
- WO-A1-2009/013869
- DE-U1- 20 121 739
- US-A- 5 225 164
- US-A- 5 792 654

## Beschreibung

Die Erfindung betrifft einen Mikroreaktor mit mindestens einer Kavität, die einen Boden, eine Seitenwandung und eine dem Boden gegenüber liegende Öffnung aufweist, wobei ein parallel zum Boden die Seitenwandung schneidender Querschnitt eine von einer runden, quadratischen oder rechteckigen Form abweichende Form hat,.

Mittels herkömmlicher Mikroreaktoren-Arrays (sogenannte Mikrotiterplatten) lassen sich z.B. 6, 24, 48, 96, 384 oder mehr einzelnen Mikroreaktoren realisieren. So wie die Anzahl der Mikroreaktoren stark variiert, kann auch das Volumen der einzelnen Reaktoren unterschiedlich sein. Während schon bei Maßstäben unter 10 ml von Mikroreaktoren gesprochen wird, kann eine weitere Reduzierung des Volumens auf unter 1 ml, unter 500 µl, unter 100 µl oder gar unter 10 µl stattfinden.

Ein Mikroreaktor dient als Reaktionsgefäß für biochemische, chemische oder enzymatische Reaktionen sowie mikrobielle Fermentationen. Ein Reaktor Array erlaubt die Untersuchung von Zellkulturen in hoher Parallelität bei geringem Arbeitsvolumen, hohem Informationsgewinn und der Möglichkeit zur vereinfachten Automatisierung. Derartige Arrays eignen sich insbesondere zur Automatisierung von Screening-Versuchen unter verbesserten Durchmischungs- und Stofftransferbedingungen und sie ermöglichen einen nach außen isolierten oder sterilen, aseptischen oder monoseptischen Betrieb.

In vielen Bereichen der Biologie, der Chemie, der Verfahrenstechnik, der Pharmazeutik und der Medizin ist ein Screening von biologischen Systemen notwendig (z.B. die Selektion von geeigneten biologischen Stämmen, Enzymen oder geeigneten Kulturmedien und -bedingungen). Dabei besteht ein Bedarf an hohen Probendurchsätzen (Parallelisierung der Versuche) und an einer Reduzierung der zum Teil teuren Ausgangsstoffe.

Mit den heute verwendeten Bioreaktoren, wie Schüttelkolben, Kleinfermentern und Reagenzgläsern ist man nicht in der Lage, diesem Bedarf gerecht zu werden. Die etablierten Techniken werden den Anforderungen an die Automatisierung, die Kostenminimierung und dem benötigten hohen Durchsatz nicht gerecht. Gerade bei biokatalytischen Systemen ist der Bedarf an vielen parallelen Versuchen im Mikrolitermaßstab besonders hoch, da solche Prozesse im Allgemeinen langsamer ablaufen und gerade in der Entwicklungsphase teurer als vergleichbare chemische Prozesse sind. Daher besteht die Notwendigkeit, Mikrobioreaktoren zu entwickeln, die auf kleinstem Raum eine geeignete Umgebung für die biologische Kultivierung und für biokatalytische Reaktionen liefern.

Als wichtige Voraussetzungen für geeignete Betriebsbedingungen sind hierbei zwei Kriterien hervorzuheben: die Möglichkeit die entsprechenden Versuche unter sterilen oder monoseptischen Bedingungen durchzuführen und die Gewährleistung eines für die biologische Kultur oder das biokatalytische Reaktionssystem geeigneten und ausreichenden Stofftransfers (flüssig-flüssig, flüssig-gas, fest-flüssig, fest-gas).

Mikroreaktoren-Arrays, wie zum Beispiel Mikrotiterplatten, bieten eine ideale Plattform, um einen hohen Parallelisierungsgrad zu erreichen. Aufgrund der kleinen Reaktionsvolumina (z.B. >10 µl bis <10 ml pro Kammer), dem hohen Parallelisierungsgrad (z.B. 6 bis 1536 Kammern pro Platte) und der Möglichkeit zur Automatisierung der Kultivierungsprozesse (von Robotern handhabbare Form) stellen Mikroreaktoren-Arrays den insgesamt kostengünstigsten und zukunftsträchtigsten Bioreaktor dar.

Weiterhin ist der Einsatz nicht-invasiver, optischer Messmethoden zur Erfassung von Prozessgrößen in diesem Reaktortyp bereits weit fortgeschritten. Zudem lassen sich die Betriebsbedingungen in geschüttelten Mikrotiterplatten hinsichtlich des Stofftransfers (maximale Sauerstofftransferkapazität, OTRmax) bereits gut charakterisieren.

Es konnte gezeigt werden, dass mit Standard-96-Well-Mikrotiterplatten (runder Querschnitt) nur maximale Sauerstofftransferkapazitäten OTRmax von 0,030 mol/l/h bei Luftbegasung erreichbar sind. (Hermann R., Lehmann M., Büchs J.: Characterization of gas-liquid mass transfer phenomena in microtiter plates. Biotechnol Bioeng, 81(2), 178-186, 2003). Dies ist jedoch für die Kulturführung von aeroben, mikrobiellen Kulturen sehr häufig nicht ausreichend, um die Kultur nicht Sauerstoff-limitiert zu führen.

Die Anforderungen vieler mikrobieller Kulturen liegen häufig über OTR-Werten von 0,05 mol/l/h, in vielen Fällen reichen sie sogar an OTR-Werte von 0,1 mol/l/h in Batch-Prozessen und sogar bis zu 0,3 mol/l/h in Feedbatch-Prozessen heran. In quadratischen und runden 96-Deep-Well-Platten konnten höhere OTRmax-Werte von 0,1-0,15 erreicht werden. Jedoch konnten diese Werte nur mit entsprechend kleinen Füllvolumina von 200 µl erreicht werden. Dabei ist die Flüssigkeit fast vollkommen abgelöst vom Boden und eine optische Messung am Boden nicht mehr möglich. Zudem existieren derzeit keine Deep-Well-Platten mit optisch transparentem Boden, was dadurch eine optische Messung durch den Boden unmöglich macht.

Aktuell werden Mikrotiterplatten bereits zum Screening von biologischen Systemen eingesetzt. Dazu werden die einzelnen Reaktionskammern befüllt, angeimpft und auf einem Rotationsschüttler inkubiert. Durch die meist orbitale Schüttelbewegung wird der Eintrag von Sauerstoff in die Reaktionsflüssigkeiten verbessert und eine Durchmischung des Reaktionsgemisches erreicht. Um das System steril zu halten, werden die Mikrotiterplatten durch eine luftpermeable Membran (Porengröße < 0,2 µm) oder eine luftdichte Folie oder eine Deckelkonstruktion abgedeckt oder offen in steriler Umgebung kultiviert.

Die für die beschriebenen Anwendungen eingesetzten Mikrotiterplatten werden heute von verschiedensten Herstellern in zwei grundsätzlichen Ausführungen angeboten: Mit kreisrunden oder mit rechteckigen Kavitäten. Die ersten Mikrotiterplatten wurden in 1951 vom Ungarn Dr. G. Takatsky gefertigt und besaßen einen runden Querschnitt. In den 90er Jahren wurden dann Mikrotiterplatten mit quadratischem und rechteckigem Querschnitt eingeführt. Mikrotiterplatten werden allerdings in sehr vielen Bereichen der Chemie, Medizin, Biotechnologie und Biologie angewendet, so dass so gut wie keine Entwicklung speziell für die Kultivierung von Zellen stattgefunden hat.

Einige der wenigen Ausnahmen beschreibt die Patentschrift US 5225164 aus dem Jahre 1991, in der die Möglichkeit beschrieben wird, in einer quadratischen Kavität an mindestens einer der vertikalen Wände der Kavität einen in die Kavität hineinragenden Stromstörer anzubringen. Diese Lösung kann grundsätzlich zu einem verbesserten Stofftransfer führen, jedoch können die Strombrecher bei starker Ausprägung das Umlaufen der Flüssigkeit in den Reaktoren, eine gute Durchmischung und einen guten Stofftransfer stark einschränken.

Andererseits können die Strombrecher zur starken Tropfenbildung/Spritzern beitragen, was zu Inhomogenitäten, verstärktem Wandwachstum und zur Benetzung und zum Verschluss der gasdurchlässigen Abdeckung der Platten führt (Büchs J., Introduction to advantages and problems of shaken cultures, Biochem. Eng. J. 7(2), 91-98, 2001). Des Weiteren ist auf dem Markt und in der Fachliteratur kein weiterer Hinweis auf die Realisierung und Untersuchung einer Mikrotiterplatte mit einer Variation der Kavitäten-Geometrie (außerhalb des kreisrunden und des rechteckigen Querschnittes) zu finden.

Aus der Praxis ist bekannt, dass der Einsatz von Mikrotiterplatten mit kreisrunden Kavitäten in einem Messsystem wie der von der Firma m2p-labs vertriebenen Bio-Lector-Technologie (WO 2005/098397), zu Problemen bei der Messwerterfassung während des Schüttelvorganges führt. Die permanente Schüttelbewegung ist notwendig, um einen kontinuierlich, guten Stofftransfer in die Reaktionsflüssigkeit zu gewährleisten, sie führt allerdings bei hohen Schüttler-Drehzahlen zur drastischen Verringerung der Flüssigkeitsschicht bis zum vollständigen Freilaufen am sensorbesetzten Boden der Kavität und somit zu Problemen bei der Messwerterfassung (s. auch: Kensy F., John G.T., Hofmann B., Büchs J., Characterisation of operation conditions and online monitoring of physiological culture parameters in shaken 24-well microtiter plates; Bioprocess and Biosystems Engineering 28(2), 75-81, 2005).

Zur Abdeckung von solchen Mikrotiterplatten sind verschiedene Systeme auf dem Markt. Zum einen wird von den meisten Herstellern von Mikrotiterplatten ein Kunststoffdeckel mitgeliefert, der lose auf die Mikrotiterplatte aufgesetzt wird.

Zum Zweiten hat sich das Konzept einer aufgeklebten Folie oder Membran am Markt durchgesetzt. Für diese Verwendung sind auch Zusatzgeräte (Sealer) für den manuellen oder automatisierten Betrieb von verschiedenen Anbietern erhältlich.

Als eine dritte Variante werden Matten aus flexiblem Kunststoff (z.B.: Silikon) vertrieben, deren noppenartige Ausstülpungen in jede einzelne Kavität greifen und sie dadurch verschließen.

Am Markt sind nur zwei Systeme bekannt, die einen formstabilen Deckel mittels einer Kraft dicht auf eine Mikrotiterplatte aufpressen. Zum einen handelt es sich dabei um ein System, welches als "Sandwich Cover Plate" von der Firma EnzyScreen vertrieben wird.

Zum Zweiten ist aus der Patentschrift US 6896848 eine Abdeckung bekannt, welche komplett um die Mikrotiterplatte herumgreift, und so einen Halt auf der Mikrotiterplatte findet. Für beide Systeme sind besondere Vorrichtungen zur Applikation oder beim Aufbringen des Deckels notwendig. Beide Systeme sind daher nicht ohne zusätzliche Halterungen oder Applikatoren automatisierbar.

Eine andere Mikrotiterplatte ist aus der EP 1 733 793 A2 bekannt.

Für einen Großteil der Anwendungen (insbesondere chem. Reaktionen mit einer Gasphase oder Zellkultivierungen) sind die nach dem Stand der Technik bekannten und oben beschriebenen Reaktionsgefäße nicht geeignet. Sie weisen folgende Nachteile auf:
- Unzureichender Gasaustausch zwischen der umgebenden Gasphase und der Flüssigphase in der Kavität
- Unzureichende oder zu langsame Mischung verschiedener Komponenten innerhalb der Kavität (Flüssig-Flüssig Mischung oder Fest-Flüssig Suspension)
- Mit steigender Schüttelfrequenz steigt die Flüssigkeit an der Wandung empor und erreicht bereits bei relativ niedrigen Schüttelfrequenzen den oberen Rand der Kavität. Dadurch wird das verwendbare Füllvolumen begrenzt, da sonst der Austritt der Flüssigkeit über den Rand der Kavität oder eine Verstopfung der aufgebrachten Abdeckungsmembran stattfinden würde.
- Freilaufen des Kavitätenbodens bei erhöhten Drehzahlen, was eine Messung mit am Boden immobilisierten oder angebrachten Sensoren oder optische Messungen in der Flüssigkeit von unterhalb des Bodens nicht zulässt.
- Die Verwendung von z.B. quadratischen Kavitätsquerschnitten oder Kavitäten mit Stromstörern kann zu Stromstörungen führen, welche die Bildung von Tropfen und/oder Aerosolen hervorrufen. Dies kann zur Abscheidung von Feststoffen und Reaktionsbestandteilen an den Wänden der Kavität sowie zur Verstopfung der Mikroreaktoren-Array-Abdeckung führen. Ebenso können optische Messungen durch die Bildung von Tropfen und/oder Aerosolen beeinträchtigt werden.
- Schaumbildung bei zu stark ausgeprägten Stromstörern.

Im Bezug auf die verschiedenen Systeme an Mikroreaktoren-Array-Abdeckungen, die gegenwärtig bekannt sind, sind vor allem folgende Nachteile als gravierend anzusehen:
- Kein fester und dichter Verschluss der Mikrotiterplatte insbesondere während einer Schüttelbewegung (nur lose aufzusetzende Standard-Deckel aus Kunststoff).
- Komplizierte Handgriffe, teilweise sind Zusatzgeräte notwendig, um eine Abdeckung auf der Mikrotiterplatte zu fixieren. Keine oder nur aufwendige Möglichkeiten und teure Lösungen der Automatisierung (Klebefolien und Kunststoffmatten).
- Keine oder nur unzureichende/inhomogene Gasversorgung und/oder unerwünscht hohe Verdampfung von Reaktionsflüssigkeit bei der Verwendung etablierter Mikrotiterplatten-Abdeckungen (Folien, Deckel-Systeme).
- Keine Möglichkeit der Beprobung oder der Flüssigkeits/Feststoff-Zugabe/Abnahme in die Mikrotiterplatte ohne Kontaminationsgefahr für die durchgeführte Reaktion, da keine Abdeckung existiert, die ohne zusätzlichen Aufwand oder ohne zusätzliche Geräte wiederverschließbar ist und gleichzeitig einen Gastransfer gewährleistet.

Aufgabe der Erfindung ist es, oben beschriebene Nachteile konventioneller Mikrotiterplatten zu überwinden und so das etablierte Konzept einer Mikrotiterplatte als Gefäß vorwiegend für chemische und biochemische Reaktionsansätze grundsätzlich hin zu einem vollwertigen und universell einsatzfähigen Reaktions- und Kultivierungssystem zu erweitern, indem vorzugsweise folgende Punkte erfüllt werden.

In Bezug auf die Mikroreaktoren-Arrays sind das:
- Intensivierter Gasaustausch mit der Flüssigkeit innerhalb einer Kavität
- Intensivierung der Durchmischung einer Flüssigkeit oder Suspension in einer Kavität
- Verhinderung des Überschwappens der Reaktionslösung aus den Reaktionsgefä-ßen bei den erforderlichen hohen Schütteldrehzahlen.
- Verhinderung des Freilaufens des Bodens einer Kavität während des Schüttelvorganges (z.B.: zur Gewährleistung einer optischen oder beliebigen Messung am Boden bzw. durch den Boden) und Realisierung eines ständigen Kontaktes zu möglichen am Boden der Kavität angebrachten Sensoren.
- Vermeidung von Tropfen- und/oder Aerosolbildungen, welche die Messungen beeinträchtigen können, Reaktionsbestandteile und/oder Biomasse an den Wänden abscheidet und/oder durch Verstopfung den Stofftransfer durch die gasdurchlässige Abdeckung des Mikroreaktoren-Arrays behindern bzw. blockieren können.
- Geringe Schaumbildung durch möglichst homogene Flüssigkeitsbewegung.

In Bezug auf die Abdeckung der Mikroreaktoren-Arrays sind das:
- Fester und dichter Abschluss jeder einzelnen Kavität gegenüber den Nachbarkavitäten und der Umgebung durch Aufbringen einer Abdeckung, die auf dem Mikroreaktoren-Array arretiert und/oder wieder gelöst werden kann.
- Einfache Handhabung der Abdeckung mit der Möglichkeit zur Automatisierung mit herkömmlichen Greifarmen von z.B. Liquid-Handling-Systemen
- Reduzierung der Verdunstung bei gleichzeitig ausreichendem Gastransfer durch Öffnungen im Deckel, durch die Anpassung der Größe dieser Öffnungen und/oder durch die Verwendung diffusionssteuernder Materialien mit denen diese Öffnungen bedeckt sind.
- Möglichkeit der sterilen Beprobung und/oder Zu- und Abfuhr von Flüssigkeiten oder Feststoffen in die einzelnen Kavitäten eines Mikroreaktoren-Arrays.

In Bezug auf einen Mikroreaktor wird die Aufgabe gelöst, indem ein parallel zum Boden die Seitenwandung schneidender Querschnitt eine von einer runden, quadratischen oder rechteckigen Form abweichende Form hat. Als Form ist hierbei die Grundform gemeint, wobei diese Grundform von kleineren Strombrechern nicht verändert wird.

Die Lösung der beschriebenen Aufgaben erfolgt durch eine Veränderung der Geometrie einer Kavität, weg von den etablierten Geometrien einer kreisrunden Zylinderform oder eines rechteckigen Querschnitts.

Die Aufgabe wird gelöst indem die nach dem Stand der Technik bekannten runden bzw. quadratischen Kavitäten so verändert werden, dass sich die positiven Eigenschaften einer Stromstörung durch das Einbringen von Vorsprüngen oder Einbuchtungen in die Kavität sowie die positiven Eigenschaften einer runden Kavität und somit einer möglichst ungestörten Strömung für den beschriebenen Anwendungsfall ideal ergänzen.

Durch die neuen vorgeschlagenen Formen der Kavitäten, wird die rundläufige Flüssigkeitsbewegung bei der Aufbringung einer orbitalen Schüttelbewegung mäßig gestört.

Durch die Störung oder Behinderung der gleichmäßigen Wandströmung bildet sich ein turbulentes Strömungsprofil aus, welches einen positiven Einfluss auf die Durchmischung sowie den Stofftransfer aus der Gasphase in die Flüssigphase und entgegengesetzt besitzt. Dabei ist die Ausbildung der Vorsprünge oder Einbuchtungen so abzustimmen, dass es nicht zur Tropfen- und Aerosolbildung kommt, die ein Verstopfen einer aufliegenden Abdeckung (z.B. einer Membran) oder das Festsetzen/Abscheiden von Flüssigkeit oder Feststoff (z.B. Biomasse) an den Reaktorwänden zur Folge haben könnte. Durch die Abstimmung der stromstörenden Wirkung kann weiterhin erreicht werden, dass die Flüssigkeit den Boden kontinuierlich benetzt und somit optische oder andere Messungen am Kavitätsboden ermöglicht werden. Ebenso kann durch die Abstimmung der stromstörenden Wirkung das mögliche Füllvolumen, bis es zu einem Überschwappen der Flüssigkeit bei einer entsprechenden Schüttelfrequenz kommt, erhöht werden.

Die Aufgabe kann durch die Realisierung verschiedener Kavitätsgeometrien gelöst werden:

Der erste Variationsansatz für die Grundfläche der Kavitäten geht aus vom einen Extrem einer quadratischen Grundfläche und nähert sich über die Zunahme der Anzahl der Ecken dem anderen Extrem einer runden Grundfläche an. Es wird daher vorgeschlagen, dass der Querschnitt mehr als vier Ecken aufweist.

Die konstruktionsrelevante Länge der Grundseite eines Vieleckes lässt sich bei gegebener Fläche von 112,16 mm² auch über die Konstruktion eines Dreiecks zwischen einer Grundseite und zwei benachbarten Radien des Vielecks berechnen. Daher wird alternativ vorgeschlagen, dass der Querschnitt weniger als vier Ecken aufweist.

Im zweiten Variationsansatz wurde, wiederum ausgehend von einem Quadrat, der Übergang zur kreisrunden Grundfläche über die Konstruktion von Kreisen mit zunehmendem Radius in den Ecken des Quadrates realisiert. Konstruktionsrelevant sind die Größen des Radius des Eck-Kreises, sowie der verbleibenden Gerade des AusgangsQuadrates.

Vorteilhaft ist es, wenn die Kavität von der Form eines Polygons abweicht.

Die Form kann damit beschrieben werden, dass ein parallel zum Boden die Seitenwandung schneidender Querschnitt mindestens ein konkaves und/oder konvexes Kreissegment aufweist, das mit einem Radius in den Querschnitt hinein ragt oder aus ihm heraus ragt, wobei dieser Radius zwischen 0,067 und 0,49 mal der Diagonalen des Querschnittes beträgt.

Ein Beispiel sieht vor, dass die Grundform des Querschnittes ein beliebiges Polygon oder ein Kreis ist, die mehrere konkave oder konvexe Kreissegmente aufweisen.

Der Querschnitt kann einen Bogen aufweisen, der ein Kreissegment von mehr als 90° bildet, oder der Querschnitt kann mehr als 3, vorzugsweise mehr als 4 Bögen aufweisen, die jeweils ein Kreissegment von mehr als 90° bilden.

Im dritten Variationsansatz wurde ein Fünfeck als Ausgangsform gewählt, und über die Abrundung der Ecken schrittweise in einen Kreis überführt.

Im Hinblick auf den zweiten und den dritten Variationsansatz wird erfindungsgemäß vorgeschlagen, dass der Querschnitt Ecken mit einem Radius von mehr als 0,5 mm aufweist.

Ein vierter Ansatz, die ursprünglich kreisrunde Grundform der Kavität abzuändern, besteht im Einbringen von Stromstörern unterschiedlicher Form und Größe. Die hierbei entstehende Grundfläche ist in vielen Fällen nicht ohne weiteres berechenbar. Um hier die Vorgabe von 112,16 mm² zu erreichen, wurde die Fläche nach dem Zeichnen mit der Software AutoCAD, Ver. 14.01 der Firma Autodesk Inc. vermessen und anschließend entsprechend skaliert.

Dabei ergibt sich eine Form, bei der der Querschnitt einen in die Kavität hineinragenden Bereich aufweist. Eine Alternative sieht vor, dass der Querschnitt einen aus der Kavität herausragenden Bereich aufweist. Vorteilhaft ist es für viele Ausführungsformen, wenn der Bereich in einer Ecke angeordnet ist.

Weiterhin wird vorgeschlagen dass mehrere derartige Bereiche mit unterschiedlichen Abmessungen vorgesehen sind oder dass mehrere derartige Bereiche aneinander angrenzen.

Im einfachsten Fall wurden rechteckige oder halbkreisförmige Schikanen über die gesamte Höhe der Kavität an ihren Wänden installiert.

Es wird daher vorgeschlagen, dass der Bereich ein Rechteck oder ein Kreissegment ist.

Weiterhin kann der jeweilige Querschnitt der verwendeten Geometrie der Kavitäten sich in Höhenrichtung weiten, um z.B. beim Spritzguss eine bessere Entformung zu gewährleisten, oder sich in Höhenrichtung verengen, um z.B. das Füllvolumen bei einer entsprechenden Schüttelfrequenz weiter erhöhen zu können, ohne dass ein Überschwappen der Flüssigkeit eintritt.

Als weitere Lösung der Aufgabe können die oben genannten Kavitätsgeometrien verwendet werden, die dann in Höhenrichtung nach oben oder nach unten in eine andere Kavitätsgeometrie übergehen. Der Übergang kann dabei zwischen einer der hier beschriebenen Kavitätsgeometrien erfolgen oder in eine runde, quadratische oder rechteckige Kavitätsgeometrie übergehen. Ebenso kann ein Übergang zwischen einer runden, quadratischen oder rechteckigen Kavitätsgeometrie erfolgen.

Als Beispiel wird daher vorgeschlagen, dass ein weiterer parallel zum Boden die Seitenwandung schneidender Querschnitt eine runde, quadratische oder rechteckige Form aufweist.

Für bestimmte Anwendungen kann es auch vorteilhaft sein, wenn mindestens ein den Querschnitt veränderndes Bauteil durch den Boden oder einen Deckel in die Kavität eingebracht ist.

Um Messungen durch den Boden zu ermöglichen wird vorgeschlagen, dass der Boden aus einem optisch transparenten Material ausgebildet ist.

Vorteilhaft ist es, wenn der Mikroreaktor mehrere Kavitäten aufweist, die besonders bevorzugt in Form eines Arrays angeordnet sind.

Unabhängig von den zuvor beschriebenen Ausführungsformen und den damit erzielbaren Vorteilen ist es vorteilhaft und erfindungswesentlich, wenn ein Mikroreaktor - insbesondere ein zuvor beschriebener Reaktor - einen speziellen Deckel aufweist.

Diese Abdeckung weist vorzugsweise eine gasdurchlässige Fläche auf, um insbesondere bei einem Array jede einzelne Kavität gegenüber Feststoffen und Flüssigkeiten aus der Umgebung abdichtet. Gleichzeitig ist es vorteilhaft, wenn oberhalb einer jeden Kavität eine Öffnung angebracht ist, die in ihrer Form und Größe, sowie dem sie verschließenden Material so gestaltet ist, dass die Verdunstung von Reaktionsflüssigkeit stark vermindert wird und ein Stofftransfer aus der umgebenden Gasphase in die Flüssigphase in der Kavität und in umgekehrte Richtung nicht beeinträchtigt wird.

Weiterhin wird vorgeschlagen, dass der Deckel eine wiederverschließbare Fläche aufweist. Besonders bevorzugt ist es, wenn der Deckel bis auf eine gasdurchlässige und/oder wiederverschließbare Fläche einstückig mit der Wandung und/oder dem Boden ausgebildet ist.

Vorteilhaft ist es, wenn der Deckel ein Sandwich-Material aus einem festen, einem flexiblen und/oder einem gasdurchlässigen Material aufweist. Eine Ausführungsform sieht vor, dass der Deckel einen stabilen Rahmen mit einer Dichtung aufweist.

Der Deckel kann unter Vorspannung an der Wandung befestigbar sein und er kann nach dem Luerprinzip an der Wandung befestigt sein. Hierbei ist bevorzugt, dass der männliche Teil eines Luerverschlusses am Deckel angebracht ist und die Wandung das weibliche Gegenstück bildet.

Eine Variante sieht vor, dass der Deckel über gegeneinander verschiebbare schiefe Ebenen mit der Wandung in Verbindung steht. Dies kann beispielsweise dadurch erreicht werden, dass zum Lösen des Deckels von der Wandung in einen Spalt ein Keil einschiebbar ist, um den Deckel von der Wandung zu lösen.

Um den Deckel abzuheben wird vorgeschlagen, dass zum Lösen des Deckels im Deckel Löcher angeordnet sind, in die ein Greifer eingreifen kann. Insbesondere hierbei ist es vorteilhaft, wenn die Greifer zum Lösen des Deckels eine Anordnung zum Aufbringen eines mechanischen oder pneumatischen Gegendrucks aufweisen. Zum Beispiel können zum Lösen eines verankerten Deckels Löcher in Öffnungen vorgesehen sein, durch die Stifte oder hohle Nadeln eines Greifarmes zum Lösen der Abdeckung von der Reaktionsgefäßanordnung einen mechanischen oder pneumatischen Druck auf die Kavität aufbringen.

Zur Befestigung des Deckels wird vorgeschlagen, dass der Deckel an der Wandung angeklebt ist. Weiterhin kann der Deckel rastend an der Wandung befestigt sein oder er kann eine Einrichtung zum Erzeugen eines Unterdrucks in der Kavität aufweisen.

Vorteilhaft ist es, wenn der Deckel eine Zu- und Abführung von Reaktionsteilnehmern und eine Beprobung ohne Unterbrechung des Schüttelvorganges ermöglicht.

Ein Ausführungsbeispiel sieht vor, dass der Mikroreaktor ein Teil eines Mikroreaktoren-Arrays mit mehreren gleichartigen Kavitäten bildet und er vorzugsweise eine Schütteleinrichtung aufweist.

Die Zeichnung zeigt Messergebnisse und Ausführungsvarianten zu Mikroreaktoren, Reaktor-Arrays und verschiedene Abdeckungen für Reaktoren und Reaktor-Arrays.

Es zeigt
- Figur 1: Variationen der Eckenanzahl einer Kavität,
- Figur 2: Variationen der Ausbildung von Ecken an einer quadratischen Kavität,
- Figur 3: Variationen der Ausbildung der Ecken an einer fünfeckigen Kavität,
- Figur 4: schematische Abbildungen von Kavitäten mit rechteckigen und halbkreisförmigen Stromstörern,
- Figur 5: schematisch die Konstruktion einer fünfeckigen Grundfläche mit asymmetrischen halbkreisförmigen Schikanen,
- Figur 6: schematisch fünf- und sechseckige Grundflächen mit asymmetrischen halbkreisförmigen Schikanen,
- Figur 7: schematisch vier-, fünf- und sechseckige Grundflächen mit abgerundeten Ecken- und Spitzenschikanen,
- Figur 8: eine Fotographie verschiedener Mikroreaktorgeometrien als Array angeordnet,
- Figur 9: eine Fotographie der Prototypen der Abdeckung mit Luer-Prinzip für Mikroreaktorenarrays,
- Figur 10: schematische Darstellungen zur Erläuterung des Luer-Prinzips,
- Figur 11: schematisch eine Anordnung zum Halten des Deckels an einem Mikroreaktorarray durch Unterdruck,
- Figur 12: schematisch die Darstellung vom Haken zum Halten des Deckels an einem Mikroreaktorenarray,
- Figur 13: schematisch Möglichkeiten zum Lösen des Deckels vom Mikroreaktorenarray,
- Figur 14: schematisch einen auf einem Mikroreaktorarray aufgesetzten Multifunktionsdeckel,
- Figur 15: graphische Darstellungen von Messergebnissen für die maximale Sauerstofftransferrate in unterschiedlichen Beispielgeometrien,
- Figur 16: graphische Darstellungen von Messergebnissen für die maximale Sauerstofftransferrate in weiteren realisierten Beispielgeometrien,
- Figur 17: graphisch dargestellt Messergebnisse für die maximale Sauerstofftransferrate bei Beispielgeometrien mit Schikanen,
- Figur 18: Messergebnisse für das maximale Füllvolumen in realisierten Beispielgeometrien und
- Figur 19: Messergebnisse für die messbare Füllhöhe in einer Kavität in realisierten Beispielgeometrien bei orbitaler Schüttelbewegung.

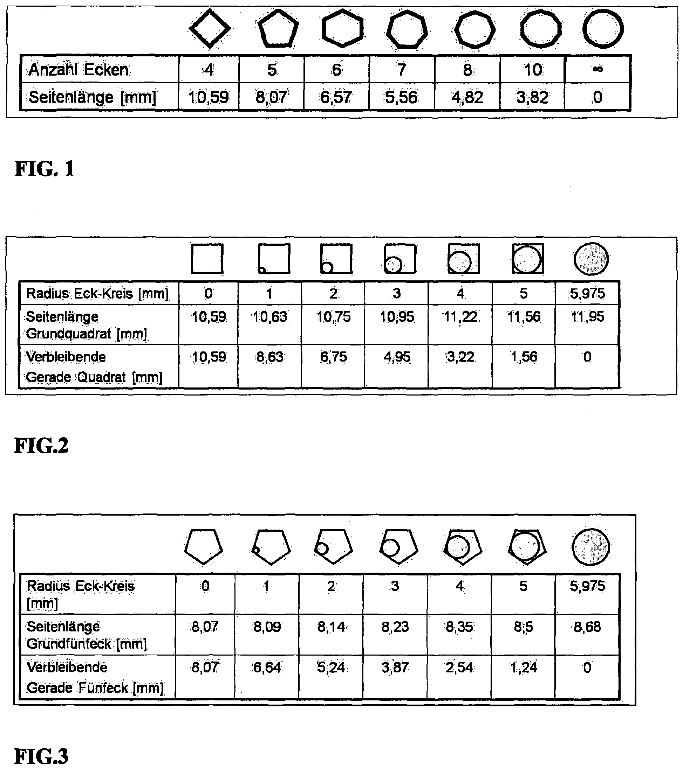

Die Figur 1 zeigt wie die konstruktionsrelevante Länge der Grundseite eines Vielecks sich bei gegebener Fläche - im vorliegenden Beispiel von 112,16 mm² - über die Konstruktion eines Dreiecks zwischen einer Grundseite und zwei benachbarten Radien des Vielecks berechnen lässt.

Der in Figur 2 gezeigte Variationsansatz geht von einem Quadrat aus, bei dem der Übergang zur kreisrunden Grundfläche über die Konstruktion von Kreisen mit zunehmendem Radius in den Ecken des Quadrates realisiert wird. Konstruktionsrelevant sind die Größen des Radius des Eckkreises sowie der verbleibenden Gerade des Ausgangsquadrates. Ihre Berechnung erfolgt über Konstruktion und Berechnung eines Drachenvielecks mit dem Mittelpunkt des Eckkreises in der Ecke des Ausgangsquadrates.

Das selbe Vorgehen am Beispiel eines Fünfecks als Ausgangsform zeigt die Figur 3.

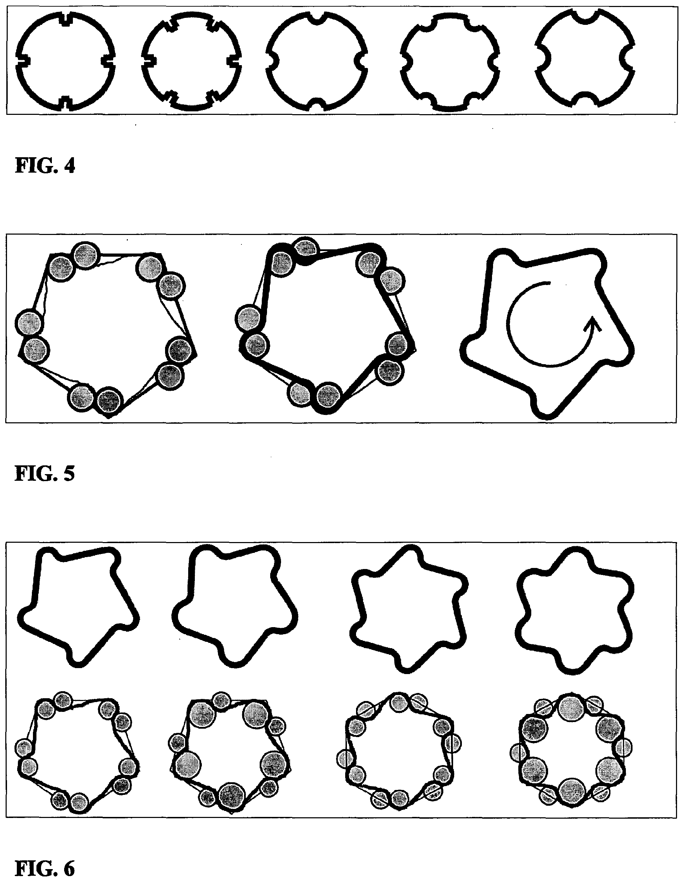

Die Figur 4 zeigt wie eine ursprünglich kreisrunde Grundform einer Kavität abgeändert werden kann, indem Stromstörer unterschiedlicher Form und Größe eingebracht werden. Die hierbei entstehende Grundfläche ist in vielen Flächen nicht ohne Weiteres berechenbar. Um die Vorgabe von 112,16 mm² zu erreichen, wurde die Fläche nach dem Zeichen mit einem CAD-System vermessen und anschließend skaliert. Hierbei wurden als Beispiele rechteckige und halbkreisförmige Schikanen über die gesamte Höhe der Kavität an ihren Wänden installiert. Dies sind jedoch nur Beispiele und sowohl die Form der Schikane als auch deren Erstreckung über die Höhe der Kavität kann in unterschiedlichen Ausführungsbeispielen variieren.

Die Figuren 5 bis 7 zeigen Grundflächengeometrien, die aus theoretischen Überlegungen zur sinnvollen Umsetzung in verschiedensten geometrischen Formen entstanden sind. Bei allen diesen Grundformen wurden Kreise definierter Größe eingebracht, deren Radius in 1 mm Schritten verändert wurde. Die Auswahl der daraus hervorgegangenen Geometrien erfolgte aufgrund einer rein theoretischen Beurteilung ihres Einflusses auf die Strömung in der Kavität. So schieden Formen mit extrem stark oder aber sehr schwach ausgeprägten Stromstörern aus der Betrachtung aus.

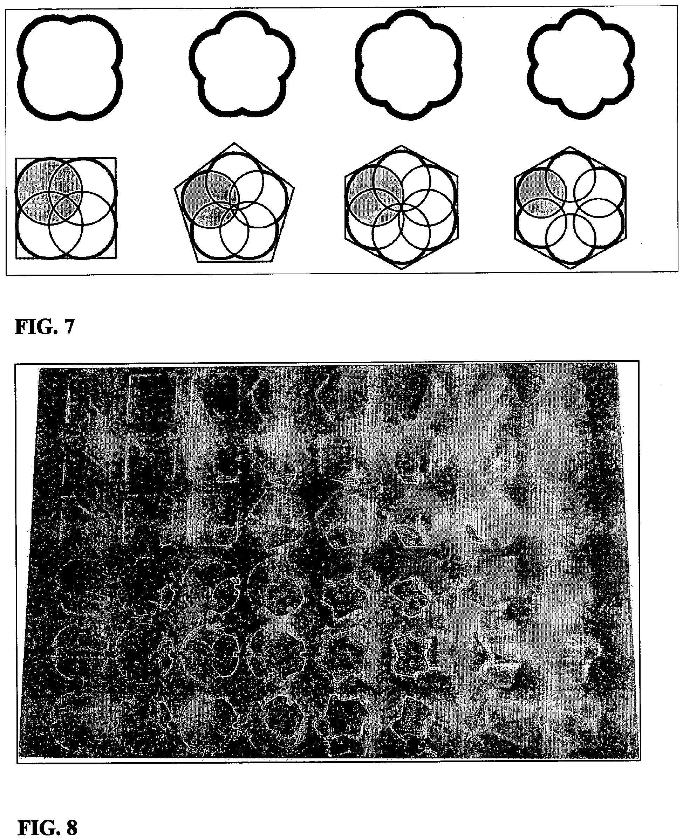

Die Abbildungen 6 und 7 zeigen Stromstörergrundflächen, die von einer fünf- oder sechseckigen Grundfläche ausgehen. Ihre Konstruktion ist in den Abbildungen 6 und 7 exemplarisch dargestellt. Die Ecke dieser Grundflächen wurden abgerundet, wobei als Radius für die Eckkreise ein bzw. zwei Millimeter genommen wurden. An jeder Ecke wird eine halbkreisförmige Schikane mit einem Radius von 1 mm angelegt. Diese Konstruktion gibt aufgrund der fehlenden Symmetrie für die Kavitäten eine Drehrichtung zum Schütteln vor.

In Figur 7 sind weitere Grundformen von Kavitäten dargestellt. Ausgehend von vier-, fünf, sechs-, oder siebeneckigen Grundformen sind die Ecken wiederum abgerundet, wobei in diesem Fall die Fläche zwischen diesen Ecken nicht flach ist, sondern eine nach innen reichende Spitze aufweist. Diese Spitzen bilden die Schikanen in diesen Kavitäten.

Das in Figur 8 gezeigte Array besteht aus unterschiedlichen Kavitäten und dient zur Untersuchung der Leistungsfunktion verschiedener Geometrien.

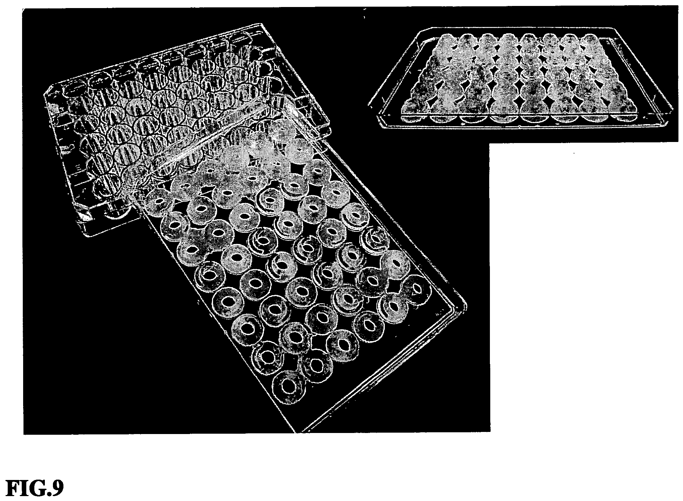

Die in Figur 9 gezeigte als Prototyp realisierte Abdeckung eines Mikroreaktorenarrays verschließt jede einzelne Kavität dicht gegen die Umgebung und hat oberhalb einer jeden Kavität eine Öffnung, die so gestaltet ist, dass die Verdunstung der Reaktionsflüssigkeit stark vermindert wird und ein Stofftransfer aus der umgebenden Gasphase in die Flüssigkeit in der Kavität und in umgekehrter Richtung nicht beeinträchtigt wird.

Dabei werden einzelne oder alle Reaktoren eines Mikroreaktorenarrays als Luer-Hülsen gegenüber dem mit Luer-Kernen gestalteten Deckeln ausgeführt. Vorteilhaft ist die Ausführung aller Kavitäten als Luer-Hülse, um gleichzeitig eine Abdichtung aller Kavitäten gegenüber der Umgebung zu erzielen.

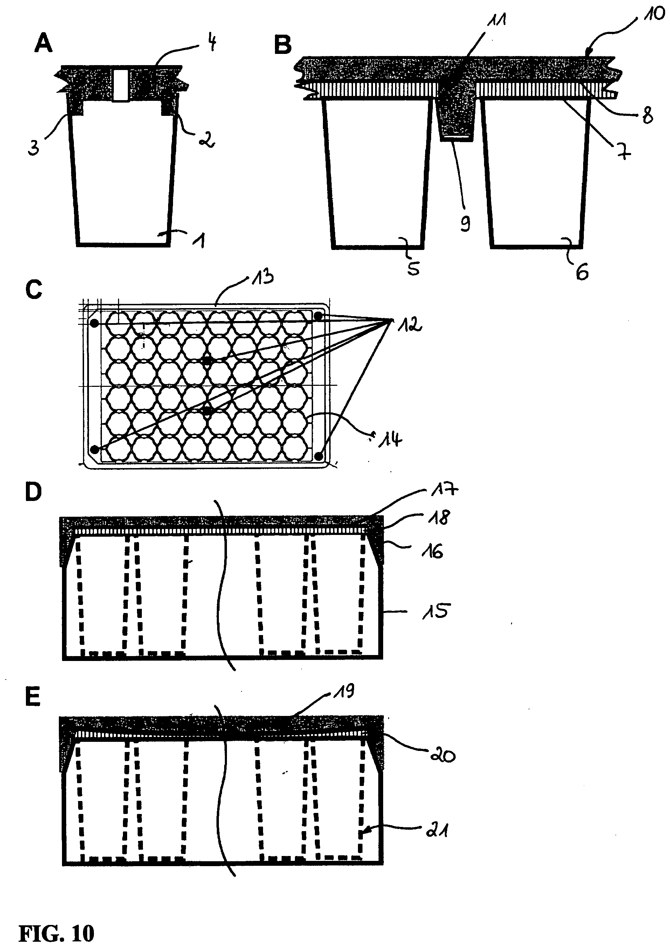

Das in Figur 10 gezeigte Luer-Prinzip hat sich bei diesem Prototypen als sehr vorteilhaft erwiesen. Es ist in der Lage, die einzelnen Reaktionsräume gegenüber der Umgebung fest und dicht zu verschließen. Hierzu ist die Kavität 1 mit einem Deckel 2, der als Abdeckung dient, verschlossen. Der Deckel 2 hat konische Passelemente 3, die an der Kavitätenwand anliegen und den Deckel 2 zur Kavität 1 abdichten. An seiner Oberseite hat der Deckel 2 eine gasdurchlässige Folie 4, die auf den Deckel aufgeklebt oder mit ihm verschweißt ist. Diese Folie sorgt für den nötigen Gasaustausch, eine reduzierte Verdampfung und den monoseptischen Betrieb. Es ist vorgesehen, die Komponenten Mikroreaktorenarray und Passungsdeckel mit abschließender gasdurchlässiger Folie vorsterilisiert dem Anwender zur Verfügung zu stellen.

Eine andere Ausführungsform ist in Figur 10B dargestellt. Hierbei ist auf den Kavitäten 5, 6 eine flexible Dichtschicht 7 vorgesehen, auf der eine gasdurchlässige Folie 8 liegt.

Der Mikroreaktorenarray hat eine konische Kavität im Korpus, die als weibliche Luer-Hülse 9 dient. Eine Abdeckung 10 für den Mikroreaktoren-Array hat einen Luer-Kern 11, der als männlicher Teil mit der Hülse 9 zusammenwirkt und die Abdeckung auf dem Array hält.

Die flexible Schicht 7 ist über den Kavitäten angebracht. Sie dichtet durch entsprechenden Anpressdruck des Deckels, der durch die Luer-Verbindung gehalten alle Wells abdichtet. Die Luer-Hülsen können beispielhaft zwischen den einzelnen Wells und auf den Rahmen des Mikroreaktoren-Arrays angebracht werden. In Abbildung 10C ist eine mögliche Ordnung gezeigt. Hierin sind die möglichen Positionen für Luer-Hülsen als Befestigungspunkte 12 auf dem Mikroreaktor-Array 13 zur Abdichtung der Kavitäten 14 gezeigt.

Die Figur 10D zeigt, wie das Luer-Prinzip über die Gesamtheit des Mikroreaktor-Arrays ausgeübt werden kann. Hierbei sind mindestens zwei gegenüberliegende Seiten des Array-Rahmens 15 angeschrägt. Sie dienen damit als Luer-Kern für den Deckel 16, der entweder eine umlaufende Luer-Hülse bildet oder nur an den überliegenden Seiten über die Seiten des Array-Rahmens 15 gestülpt ist. Der Array-Deckel 16 ist somit als Luer-Hülse ausgebildet und geht einen Kraftschluss mit dem Array-Rahmen 15 ein. Zwischen dem Deckel 16 und dem Mikroreaktor-Array-Rahmen ist wiederum eine gasdurchlässige Folie 17 und eine flexible Deckschicht 18 vorgesehen.

Um einen gleichmäßigen oder ausreichenden Anpressdrucks des Deckels 19 auf die flexible Schicht 20 an jeder Stelle des Mikroreaktoren-Arrays 21 zu gewährleisten, ist es vorteilhaft, den Deckel 19 nach innen hin auszubauchen, wie dies in Figur 10E gezeigt ist. Dadurch wird für eine gleichmäßige Spannungsverteilung gesorgt.

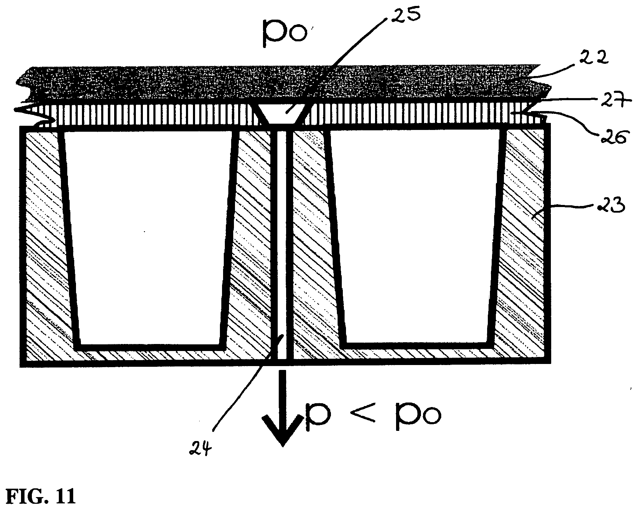

Die Figur 11 zeigt eine Ausführungsvariante, bei der der Deckel 22 auf dem Mikroreaktoren-Array 23 mit Vakuum oder Unterdruck angesaugt wird. Hierzu wird durch eine Bohrung 24 im Korpus des Mikroreaktoren-Arrays 23 Vakuum gesaugt und damit Unterdruck erzeugt und der Deckel durch einen Saugnapf oder ein ähnliches Zwischenstück 25 an den Mikroreaktoren-Array 23 gezogen. Dadurch wird die flexible Schicht 26 mit der darauf liegenden gasdurchlässigen Folie 27 auf die Kavitäten gepresst, um diese abzudichten. Die Pressung und Dichtung ist solange aktiv, wie Vakuum gezogen bzw. Unterdruck erzeugt wird. Daher ist diese Verbindung sehr leicht zu lösen. Die Saugknöpfe 25 und die Bohrungen 24 können je nach Anwendungsfall beliebig auf dem Array verteilt sein.

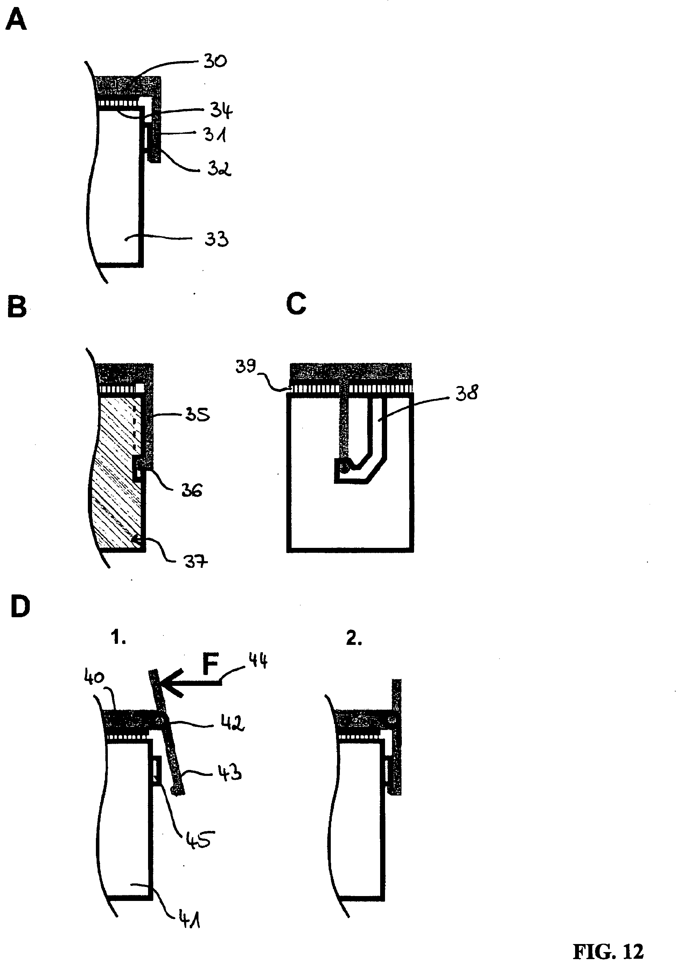

In Figur 12A ist dargestellt wie der Deckel 30 durch einen Widerhacken 31 an der Platten-Geometrie befestigt ist. Im Ausführungsbeispiel hakt sich der Widerhaken 31 an einem Steg 32 fest. Hierbei ist es lediglich erforderlich, den Deckel 30 mit seinem Widerhaken 31 auf den Mikroreaktoren-Array 33 von oben oder von der Seite aufzuschieben. Im geschlossenen Zustand wird der Deckel 30 so nah an dem Mikroreaktoren-Array 33 gehalten, dass er die flexible Schicht 34 auf die Kavitäten (nicht gezeigt) presst und somit abdichtet.

Figur 12B zeigt eine Variante, bei der der Widerhaken 35 in einer. Nut 36 im Mikroreaktoren-Array 37 festhakt.

Um den Deckel 30 später wieder zu lösen, ist es vorteilhaft, den Widerhaken 35 des Deckels 30 in einer Führungsnut 38 zu führen. In der Endlage der Führungsnut 38 wird der Widerhaken 35 durch die Federspannung der flexiblen Schicht 39 gehalten. Zur Ein- und Ausrastung in die Endlage muss eine äußere Kraft auf den Deckel 30 mittels eines Greifarmes eines Pipettierroboters oder manuell aufgebracht werden, die die Federspannung der flexiblen Schicht 38 leicht übersteigt.

Die Figur 12D zeigt eine alternative Lösung zur Befestigung des Deckels 40 auf dem Mikroreaktoren-Array 41 durch das Aufbringen einer Federspannung mittels der Feder 42 auf den Widerhaken 43. Nur durch das Aufbringen einer äußeren Kraft 44 mittels eines Greifarms eines Pipettierroboters oder manuell kann der Spannung der Feder 42 entgegengewirkt werden und der Widerhaken 43 aufgespreizt werden. Durch Verfahren des Deckels 40 nach unten und anschließender Entlastung der Feder 42 kann der Deckel 40 aufgesetzt werden und der Widerhaken an Seitensteg 45 einrasten.

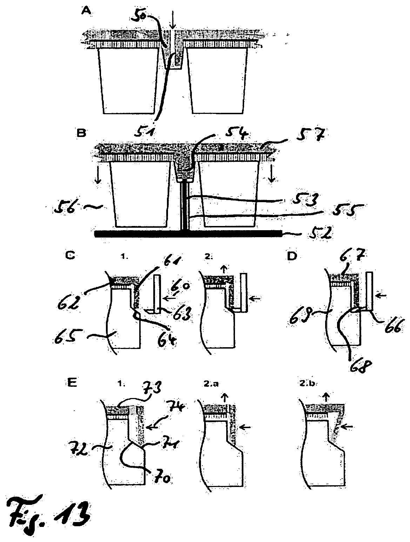

Bei einigen Applikationen kann es notwendig werden, dass der Deckel wieder von dem Mikroreaktoren-Array gelöst wird. Auch diese Möglichkeit soll mit einfachen Mitteln gelöst werden, ohne große zusätzliche Geräte neben einem einfachen Liquid-Handling-Systems mit Greifern (Standart-Pipettierroboter). Eine Möglichkeit hierzu zeigt die Figur 13. Zum einen ist es möglich, über einen Überdruck im Inneren der Luer-Hülse wie in Figur 13A gezeigt, die Luer-Verbindung zu lösen. Der Überdruck kann durch eine am Greifarm eines Pipettierroboters angebrachte Druckluftleitung durch die Bohrung im Luer-Kern appliziert werden. Dazu muss zu jedem Luer-Kern 50 eine Druckluftleitung 51 geführt werden.

Die Figur 13B zeigt, wie auf einer Platte 52 Stifte 53 angebracht sind, die die Luer-Kerne 54 von unten her losdrücken. Die Platte wird einfach auf die Stifte 53 aufgesetzt und schon drücken die Stifte 53 geführt durch Bohrungen 55 im Mikroreaktoren-Array-Grundkörper 56 den Deckel 57 an den Luer-Kernen 54 nach oben.

Wenn mehrere Luer-Verbindungen oder andere Kraftverbindungen über den Mikroreaktoren-Array zur Befestigung des Deckels angewendet werden, ist es von Vorteil, wenn nicht an jeder einzelnen Verbindung gezielt eine Kraft angewendet werden muss. Eine Lösung hierfür zeigt die in Figur 13C dargestellte Konstruktion. Hierbei wird die Kraft 60 nur auf den äußeren Rahmen 61 des Deckels 62 aufgebracht, in dem angeschrägte Schuhe 63 als Finger eines Greifarms horizontal unter den Deckel 62 gefahren werden. An der schrägen Wand 64 des Mikroreaktoren-Arrays 65 kommt es schließlich zu einer Umlenkung der Kraft 60 in die Vertikale, indem der Schuh 63 unter den Deckel 62 fährt und ihn anhebt. Die Bewegung des Schuhs 63 erzwingt eine Relativbewegung des Mikroreaktoren-Arrays 65 zum Deckel 62 und löst ihn so.

Eine leichte Abwandlung von diesem Prinzip zeigt die Figur 13D. Hier ist der Schuh 66 nach oben hin angefast. Der Deckel 67 fährt über eine Schräge 68 nach oben, wobei der Mikroreaktoren-Array 69 in seiner festen Position verharrt.

Ein weiterer Lösemechanismus ist in Figur 13E dargestellt. Dieser Lösemechanismus kommt ohne einen speziellen abgeschrägten Schuh an den Fingern eines Greifarms aus. Durch die Realisierung zweier spiegelbildlicher Schrägen 70 und 71 am Mikroreaktoren-Array 72 und der Abdeckung 73 wird beim Aufbringen einer horizontalen Kraft 74 auf die an dieser Seitenfläche teilweise flexibel ausgestaltete Abdeckung eine Umleitung dieser Kraft 74 in die Vertikale nach dem oben beschriebenen Prinzip erreicht.

In zunehmendem Maße wird es erforderlich, nicht nur online-Informationen aus den Reaktoransätzen eines Mikroreaktoren-Arrays durch entsprechenden Einsatz von Sensorik in oder an den Reaktoren zu erhalten, sondern auch die zeitlich wechselnden Reaktionszustände mit weiterer offline-Analytik zu verifizieren oder weitere Einblicke zu erlangen. Daher ist es wichtig, auch während der Reaktionsführung aus den einzelnen Reaktoren Probematerial zu erhalten und die Reaktionen im Anschluss daran unbeeinflusst weiter zu betreiben. Es kann auch notwendig sein, flüssige oder feste Substanzen dem Reaktionsgemisch zu- oder abzuführen. Daher ist es erforderlich, einen reversiblen Zugang zu dem Reaktionsgefäß zu erlangen. Aus diesem Grunde soll zusätzlich zu der Öffnung im Deckel für die gasdurchlässige Schicht eine Öffnung zur Beprobung und/oder der Zu- und Abfuhr von Substraten/Reaktionsteilnehmern realisiert werden.

Hierfür ist die Verwendung eines Septenmaterials aus Silikon oder einem anderen flexiblen Polymer, welches aufgrund seiner Materialeigenschaften sich von selbst wieder verschließt, vorgesehen. Mit einer Kanüle 80, einer Pipette oder einer Pipettenspitze soll die Septe schließlich durchstechbar sein, um aus dem Kavitätsinneren Probenmaterial 81 hinein oder heraus zu pipettieren. Nach dem Abzug der Kanüle 80 und dem Wiederverschluss der Septe soll die Reaktion unbeeinflusst weiter laufen. Insbesondere bei zellulären Anwendungen ist die sterile Beprobung außerordentlich wichtig, um keine Kontamination der meist monoseptischen Kulturführung zuzulassen.

Vorteilhaft ist es, insbesondere bei sehr schnellen Reaktionen und Fermentationen die Beprobung bzw. den Eingriff in den Prozess ohne Unterbrechung des Schüttelns durchzuführen, um den Stofftransport nicht zu limitieren. Hierfür ist vorgesehen, den Schüttelprozess mit einem derart kleinen Durchmesser durchzuführen, der es erlaubt auch während des Schüttelprozesses durch eine Septe in den Reaktionsraum einzudringen und eine Probe zu ziehen oder Substrate zu- oder abzuführen. Vorteilhaft sind hierbei Schütteldurchmesser von 1 bis 5 mm. Außerdem ist darauf zu achten, dass die Kanüle für die Beprobung während der Schüttelbewegung höchstens im elastischen Bereich deformiert wird oder flexibel gelagert ist.

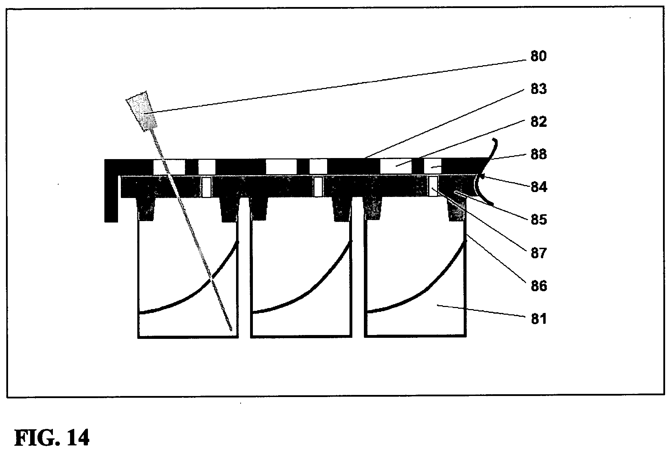

Die in Figur 14 gezeigte Anordnung weist eine Kanüle 80 auf, die durch eine Öffnung 82 in einem starren Deckelrücken 83 durch eine gasdurchlässige Folie 84 und eine flexible Deckschicht 85 in eine Kavität 86 geführt wird, um aus der Kavität 86 Reaktionsflüssigkeit zu entnehmen oder in die Kavität einzuführen. Für den Stofftransport zwischen innen und außen sind Bohrungen 87 in der flexiblen Deckschicht 85 vorgesehen, die mit Öffnungen 88 im starren Deckelrücken 83 fluchten.

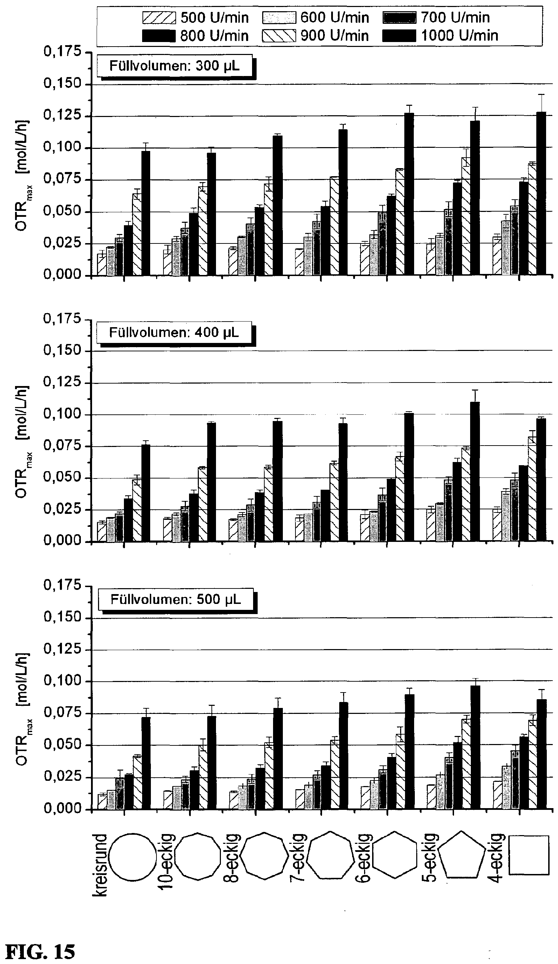

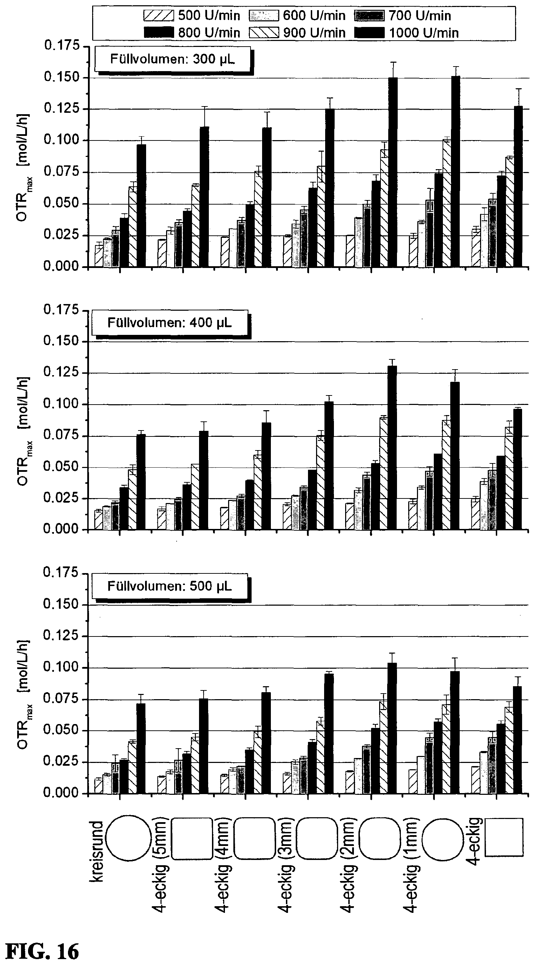

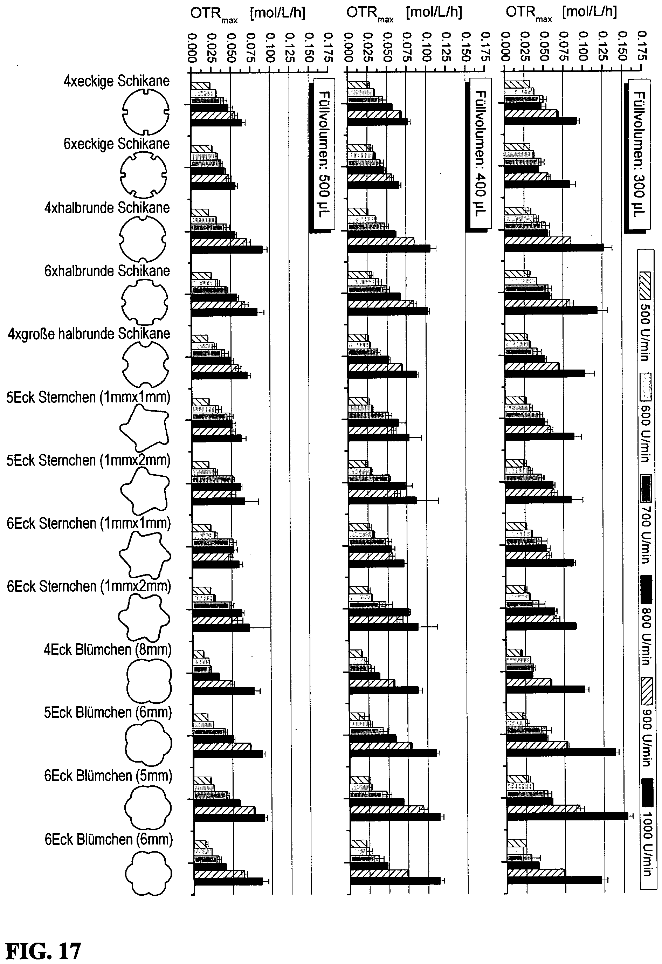

Die enormen Vorteile einer veränderten Geometrie der Kavitäten eines Mikroreaktoren-Arrays konnten in umfangreichen Versuchen mit mehreren Prototypen nachgewiesen werden. Zum Einen konnte gezeigt werden, dass eine enorme Steigerung der Sauerstofftransferrate in die Reaktionsflüssigkeit, deren Höhe insbesondere für mikrobielle Fermentationen in Mikrotiterplatten nach dem Stand der Technik einen limitierenden Faktor darstellt, erreicht werden kann. Es konnte weiterhin gezeigt werden, dass die nach dem Stand der Technik in verfügbaren Mikrotiterplatten bzw. Deep-Well-Platten verwendeten Kavitäten-Geometrien (kreisrund oder quadratisch) in keinster Weise das Optimum für den Sauerstoffeintrag darstellen. In den Figuren 15 bis 17 sind die maximalen Stofftransferraten (hier Sauerstofftransferrate; OTR (oxygen transfer rate)) bei verschiedenen Geometrien und Schütteldrehzahlen sowie unterschiedlichen Arbeitsvolumina dargestellt (Schütteldurchmesser jeweils 3 mm Orbit). Aus diesen Messdaten kann zusätzlich abgeleitet werden, dass die in der Patentschrift US 5225164 beschriebene Kavitätenform ebenfalls nicht optimal sein kann, da durch die Kombination der quadratischen Grundform mit eckigen Stromstörern kein maximaler Sauerstoffeintrag zu erwarten ist, dem gegenüber aber eine starke Tropfenbildung beobachtet werden kann.

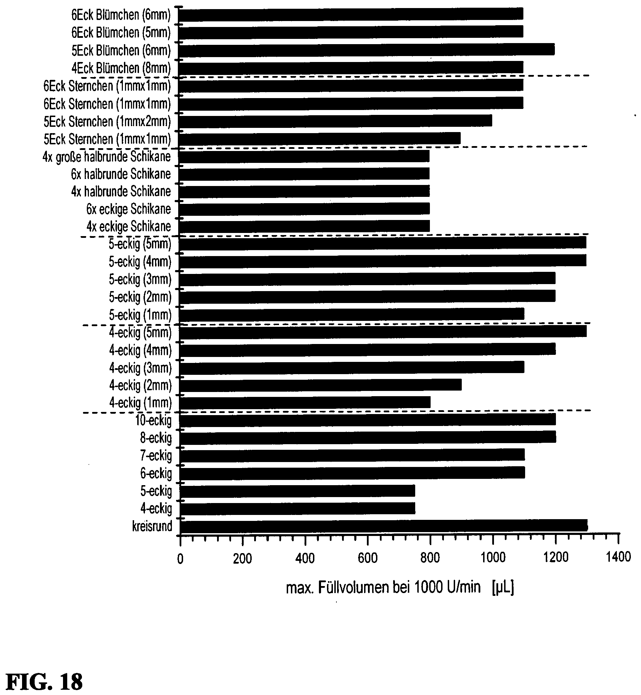

In einer zweiten Versuchsreihe wurden das Überschwappen bzw. die Bildung von Tropfen während einer orbitalen Schüttelbewegung untersucht. Für jede der realisierten Ausführungen wurde untersucht, wie hoch das maximale Füllvolumen bei einer Schüttelgeschwindigkeit von 1000 U/min und einem Schütteldurchmesser von 3 mm in einem 20 mm hohen Prototypen-Mikrotiterplatte ist (Figur 18).

Es kann gezeigt werden, dass ein vorteilhaftes Verhalten der Flüssigkeit entsteht, wenn die als Strombrecher wirkenden Vorsprünge oder Einstülpungen nur klein sind, bzw. moderate Steigungen aufweisen.

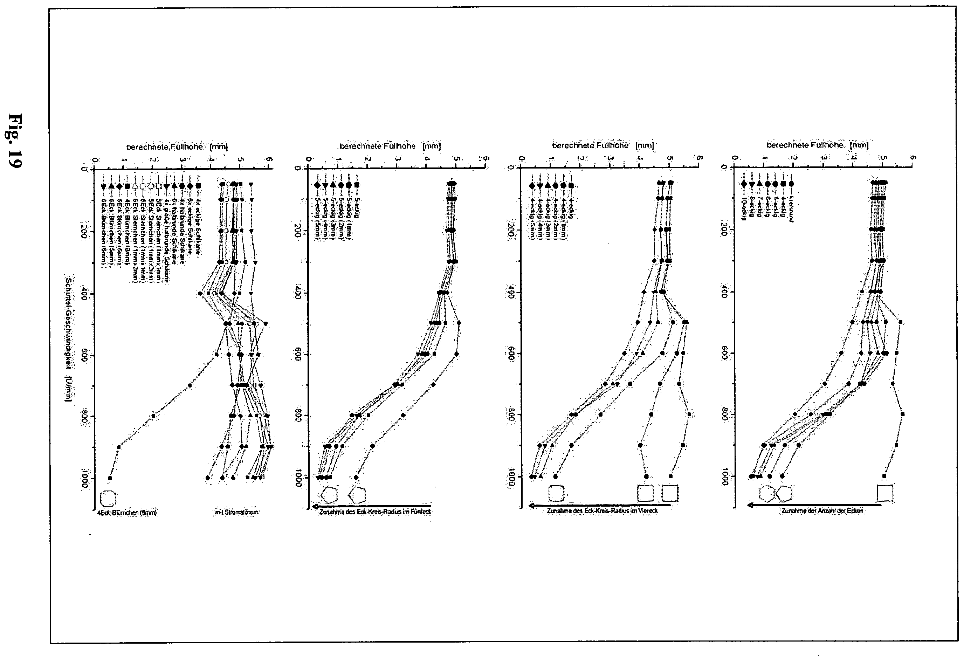

Neben diesen beiden Vorteilen der Erfindung, eines erhöhten Stofftransfers bei gleichzeitig geringer Tendenz zur Tropfenbildung bzw. zum Überschwappen der Reaktionsflüssigkeit, liegt ein weiterer wesentlicher Vorteil der Erfindung in der Verhinderung des Freilaufens des Bodens der Kavität. Um diese Eigenschaft zu untersuchen, wurde über die Fluoreszenz-Intensität einer Fluorescein-Lösung auf die am Boden eines Wells befindliche Schichtdicke der Flüssigkeit während der Rotation des Schüttlers zurück gerechnet. Die in Figur 19 beschriebenen Ergebnisse zeigen, dass je stärker die Ecken und Kanten ausgeprägt sind, desto mehr wird die Flüssigkeit daran gehindert, über die Kavitäten-Wände zu expandieren und somit vom Boden zu weichen.

Die gleichzeitige Betrachtung der drei beschriebenen Testreihen (Sauerstoffeintrag, maximales Füllvolumen, Freilaufen des Bodens) lässt erkennen, dass die Erfindung gegenüber dem Stand der Technik das Konzept der Mikrotiterplatte insbesondere bei deren Anwendung als Zellkultivierungssystem grundlegend verbessert. Dadurch, dass ein deutlich höherer Sauerstoffeintrag erzielt werden kann, bei gleichzeitig ausreichend homogener Hydrodynamik ohne Tropfen- und Spritzerbildung, ist eine in weiten Bereichen unlimitierte Fermentation von Mikroorganismen und höheren Zellen (Pflanzen-, tierische und menschliche Zellen) möglich. Durch ein Verhindern des Freilaufens des Bodens der Kavität bleibt auch bei hohen Schüttelgeschwindigkeiten eine ausreichend hohe Flüssigkeitssäule am Boden der Kavität. Dadurch wird die Flüssigkeit einer Messung am Boden der Kavität deutlich besser zugänglich gemacht. Hier angebrachte Sensoren laufen nicht Gefahr, den Kontakt zum Reaktionsgemisch zu verlieren.

Die neuartige Gestaltung der Abdeckung der Mikrotiterplatte überwindet einen gravierenden Nachteil, der normalerweise bei der Kultivierung in Mikrotiterplatten auftritt. Der insbesondere bei höheren Kultivierungstemperaturen auftretende Flüssigkeitsverlust aus der Kavität wird deutlich vermindert. Gleichzeitig wird ein ausreichender Gasaustausch zwischen Umgebung und Reaktionsvolumen ermöglicht. Durch ein Septum bleibt jede Kavität der Probennahme zugänglich. Durch den Aufbau der Abdeckung aus einem formstabilen Teil und einem flexiblen Material zur Abdichtung sowie der Möglichkeit zum leichten Lösen der Abdeckung bietet die Erfindung die Möglichkeit zur Einpassung des Systems in automatisierte Systeme (Pipettierroboter; Greifarm) ohne großes zusätzliches Equipment oder Sonder-Applikatoren.

## Patentansprüche

1. Mikroreaktor mit mindestens einer Kavität, die einen Boden, eine Seitenwandung und eine dem Boden gegenüber liegende Öffnung aufweist, wobei ein parallel zum Boden die Seitenwandung schneidender Querschnitt eine von einer runden, quadratischen oder rechteckigen Form abweichende Form hat, ***dadurch gekennzeichnet, dass*** die Grundform des Querschnittes ein beliebiges Polygon ist, das mindestens ein oder mehrere konkave oder konvexe Kreissegmente aufweist, wobei an den Ecken des Polygons Kreise mit einem Radius von mehr als 0,5 mm konstruiert sind..

2. Mikroreaktor nach Anspruch 1, ***dadurch gekennzeichnet, dass*** der Querschnitt mehr als 3, vorzugsweise mehr als 4 Bögen aufweist, die jeweils ein Kreissegment von mehr als 90° bilden.

3. Mikroreaktor nach einem der vorhergehenden Ansprüche, ***dadurch gekennzeichnet, dass*** der Querschnitt einen in die Kavität hineinragenden oder aus der Kavität herausragenden Bereich aufweist und mehrere derartige Bereiche mit unterschiedlichen Abmessungen vorgesehen sind.

4. Mikroreaktor nach einem der vorhergehenden Ansprüche, ***dadurch gekennzeichnet, dass*** ein weiterer parallel zum Boden die Seitenwandung schneidender Querschnitt eine runde, quadratische oder rechteckige Form aufweist.

5. Mikroreaktor nach einem der vorhergehenden Ansprüche, ***dadurch gekennzeichnet, dass*** mindestens ein den Querschnitt veränderndes Bauteil durch den Boden oder einen Deckel in die Kavität eingebracht ist.

6. Mikroreaktor nach einem der vorhergehenden Ansprüche, ***dadurch gekennzeichnet, dass*** der Boden aus einem optisch transparenten Material ausgebildet ist, welches Messungen durch den Boden ermöglicht.

7. Mikroreaktor nach einem der vorhergehenden Ansprüche, ***dadurch gekennzeichnet, dass*** er mehrere Kavitäten aufweist.

8. Mikroreaktor nach einem der vorhergehenden Ansprüche, ***dadurch gekennzeichnet, dass*** er einen Deckel aufweist.

9. Mikroreaktor nach Anspruch 10, ***dadurch gekennzeichnet, dass*** der Deckel eine gasdurchlässige Fläche aufweist.

10. Mikroreaktor nach einem der Ansprüche 10 oder 11, ***dadurch gekennzeichnet, dass*** der Deckel eine wiederverschließbare Fläche aufweist.

11. Mikroreaktor nach einem der vorhergehenden Ansprüche, ***dadurch gekennzeichnet, dass*** er ein Teil eines Mikroreaktoren-Arrays mit mehreren gleichartigen Kavitäten bildet.

12. Mikroreaktor nach einem der vorhergehenden Ansprüche, ***dadurch gekennzeichnet, dass*** er eine Schütteleinrichtung aufweist.

13. Mikroreaktor nach einem der vorhergehenden Ansprüche, ***dadurch gekennzeichnet, dass*** er eine Zu- und Abführung von Reaktionsteilnehmern und eine Beprobung ohne Unterbrechung eines Schüttelvorganges ermöglicht.

## Claims

1. A microreactor having at least one cavity, which is provided with a bottom, a side wall and an opening disposed opposite the bottom, wherein a cross-section intersecting the side wall parallel to the bottom has a shape which differs from a round, square or rectangular shape, ***characterized in that*** the cross section has an arbitrary polygonal basic shape which is provided with at least one or more concave or convex segments of a circle, wherein circles with a radius of more than 0.5 mm are constructed at the corners of the polygon.

2. The microreactor as claimed in claim 1, ***characterized in that*** the cross section is provided with more than 3, preferably more than 4 arcs which respectively form a segment of a circle of more than 90°.

3. The microreactor as claimed in one of the preceding claims, ***characterized in that*** the cross section has a region which protrudes into or out of the cavity and a plurality of said regions with different dimensions are provided.

4. The microreactor as claimed in one of the preceding claims, ***characterized in that*** a further cross section intersecting the side wall parallel to the bottom is provided with a round, square or rectangular shape.

5. The microreactor as claimed in one of the preceding claims, ***characterized in that*** at least one component which modifies the cross section is introduced into the cavity through the bottom or through a cover.

6. The microreactor as claimed in one of the preceding claims, ***characterized in that*** the bottom is constructed from an optically transparent material which enables measurements to be taken through the bottom.

7. The microreactor as claimed in one of the preceding claims, ***characterized in that*** it is provided with a plurality of cavities.

8. The microreactor as claimed in one of the preceding claims, ***characterized in that*** it is provided with a cover.

9. The microreactor as claimed in claim 10, ***characterized in that*** the cover is provided with a gas-permeable area.

10. The microreactor as claimed in claim 10 or claim 11, ***characterized in that*** the cover is provided with a resealable area.

11. The microreactor as claimed in one of the preceding claims, ***characterized in that*** it forms a part of a microreactor array with a plurality of identical cavities.

12. The microreactor as claimed in one of the preceding claims, ***characterized in that*** it is provided with a shaking device.

13. The microreactor as claimed in one of the preceding claims, ***characterized in that*** it authorizes the supply and withdrawal of reagents and sampling without interruption to a shaking process.

## Revendications

1. Microréacteur doté d'au moins une cavité qui présente un fond, une paroi latérale et une ouverture opposée au fond, dans lequel une section transversale coupant la paroi latérale parallèlement au fond à une forme différant d'une forme ronde, quadratique ou rectangulaire, ***caractérisé en ce que*** la forme de base de la section transversale est un polygone quelconque qui présente au moins un ou plusieurs segments de cercle concaves ou convexes, des cercles d'un rayon de plus de 0,5 mm étant réalisés aux angles du polygone.

2. Microréacteur selon la revendication 1, ***caractérisé en ce que*** la section transversale présente plus de 3, de préférence plus de 4 arcs qui constituent respectivement un segment de cercle de plus de 90°.

3. Microréacteur selon une des revendications précédentes, ***caractérisé en ce que*** la section transversale présente une zone dépassant dans la cavité ou dépassant hors de la cavité et que plusieurs zones de ces types à dimensions différentes sont prévues.

4. Microréacteur selon une des revendications précédentes, ***caractérisé en ce qu***'une autre section coupant la paroi latérale parallèlement au fond présente une forme ronde, quadratique ou rectangulaire.

5. Microréacteur selon une des revendications précédentes, ***caractérisé en ce qu'***une pièce à section transversale variable est intégrée par le fond ou par un couvercle dans la cavité.

6. Microréacteur selon une des revendications précédentes, ***caractérisé en ce que*** le fond est constitué d'un matériau optiquement transparent qui permet des mesures à travers le fond.

7. Microréacteur selon une des revendications précédentes, ***caractérisé en ce qu***'il présente plusieurs cavités.

8. Microréacteur selon une des revendications précédentes, ***caractérisé en ce qu***'il présente un couvercle.

9. Microréacteur selon la revendication 10, ***caractérisé en ce que*** le couvercle présente une surface perméable au gaz.

10. Microréacteur selon une des revendications 10 ou 11, ***caractérisé en ce que*** le couvercle présente une surface refermable.

11. Microréacteur selon une des revendications précédentes, ***caractérisé en ce qu***'il constitue un élément du réseau de microréacteurs doté de plusieurs cavités similaires.

12. Microréacteur selon une des revendications précédentes, ***caractérisé en ce qu***'il présente un dispositif de secouage.

13. Microréacteur selon une des revendications précédentes, ***caractérisé en ce qu***'il permet un apport et une évacuation de participants à la réaction et un essai sans interrompre une opération de secouage.
